# EUROPEAN PATENT SPECIFICATION

(11) **EP 3 076 300 B1**
(45) Date of publication and mention of the grant of the patent: **29.08.2018**
(21) Application number: 13898169.1
(22) Date of filing: 24.12.2013
(51) Int. Cl.: G06F 11/32, G06F 11/07, G06F 19/00

(54) **METHOD FOR MONITORING SOFTWARE CRASH AND MEDICAL EXTERNAL APPARATUS USING THE SAME**
VERFAHREN ZUR ÜBERWACHUNG VON SOFTWAREABSTÜRZEN UND EXTERNE MEDIZINISCHE VORRICHTUNG DAMIT
PROCÉDÉ DE SURVEILLANCE DU BLOCAGE DE LOGICIEL ET APPAREIL MÉDICAL EXTERNE L'UTILISANT

(30) Priority: 29.11.2013 CN 201310620050
(43) Date of publication of application: 05.10.2016
(73) Proprietor: Edan Instruments, Inc., Shenzhen, Guangdong 518067 (CN)
(72) Inventor: CHEN, Aijun, Shenzhen Guangdong 518067 (CN); ZHENG, Ruhai, Shenzhen Guangdong 518067 (CN); ZHOU, Hongwei, Shenzhen Guangdong 518067 (CN)
(74) Representative: dompatent von Kreisler Selting Werner - Partnerschaft von Patent- und Rechtsanwälten mbB
(86) International application number: PCT/CN2013/090280
(87) International publication number: WO 2015/078073

(56) References cited:
- EP-A1- 2 001 188
- CN-A- 1 755 644
- CN-A- 1 787 410
- CN-A- 101 587 449
- CN-A- 103 690 170
- US-A1- 2005 283 198
- US-A1- 2011 009 061
- US-A1- 2011 213 261
- US-A1- 2012 088 983

## Description

### TECHNICAL FIELD

The present invention relates to the technical field of medical monitoring, in particular to a method for monitoring software crash and a medical external equipment and a medical host equipment using the method.

### BACKGROUND

Currently medical electronic equipment is installed a variety of computer software systems, and if the software system crashes, it will bring huge risks to the hospital and patients. For example: the central station system served as the central system for monitoring the patients, constantly deals with monitoring data of patients, if the central station system crashes, the central station system will not be able to process patients' alarm data, also cannot store patients' monitoring data, there exists huge risk. Therefore, the central station system crash happens accidentally at any time. System crash may be caused by the software itself, and also may be caused by the operating system on which the software runs. Therefore, it needs effective method and device for monitoring software crash. In addition, other various systems of medical monitoring equipment, such as patient monitor, fetal monitor, ECG testing equipment and so on, also constantly monitor patient's various physiological parameters. If the system crashes can be found in time, the system of the monitor will not be able to make timely alarm that the patient's life is in danger. Therefore, the medical equipment system needs effective method and device for monitoring software crash, thereby solving the harm and the loss brought by the system crash.

There are many ways to monitor the system crash, such as the general watchdog technology. Using the software watchdog, the system crash caused by the crash of operating system on which the software runs is unable to be monitored, because if the operating system crashes, the software watchdog can't operate normally, the effect of watchdog can not be reached. Hardware watchdog can monitor the software crash, and can monitor the crashes caused by a variety of software. Using the hardware watchdog needs to add a hardware watchdog circuit on the circuit board, because each device needs to add the watchdog circuit, the design complexity of the device and manufacturing cost increase, which can't facilitate the convenient crash monitoring for the medical monitoring equipment system currently concerned by the medical care personnel under the situation of low design and manufacturing cost, which lacks mobility. Therefore, the existing technology has defects, which needs to be solved urgently.

Document US2012/0088983 constitutes relevant prior art to the invention because it discloses a method for monitoring an implantable medical device (IMD). It comprises the steps of: selecting a medical host equipment for collecting and detecting patient's physiological parameters, and starting up a medical equipment host system; judging whether there is an access from an external equipment by the medical equipment host system and judging whether the accessing external equipment is the external equipment for monitoring the IMD; sending state information of the IMD to the external equipment in a predetermined time interval by the medical equipment host; and determining whether the state information is received by the external equipment for monitoring said IMD.

### SUMMARY

In order to overcome the defects mentioned above, the present invention aims at providing a method for monitoring software crash and a medical external equipment and a medical host equipment using the method.

According to the invention, there is proposed a method for monitoring software crash according to claim 1, a medical external equipment according to claim 8 having a function of monitoring software crash mentioned in method claim 1, and a medical host equipment according to claim 10 having a function of monitoring software crash mentioned in method claim 1.

Dependent claims relate to preferred embodiments of the present invention.

The technical schemes of the present invention help the system of the medical equipment monitor a system crash, and generate a crash alarm prompt tone to remind the user that the system crashes if the system of the medical equipment crashes. The user can find the system crash in time, thereby reducing the risk and loss caused by the system crash. Meanwhile, since a plug-and-play external equipment for monitoring the system crash is used, the external equipment can access quite conveniently to various medical equipments to be monitored as long as the system of the medical equipment communicates according to a protocol established by the equipment for monitoring. The equipment for monitoring can access to various universal peripheral interfaces such as: USB interface, serial port, parallel port, PS2, network interface etc., and can be used after access. By using the equipment for monitoring, the user can conveniently monitor the medical monitoring equipment that the user desires to monitor, which saves the design resources and costs.

### BRIEF DESCRIPTION OF THE DRAWINGS

For ease of illustration, the present invention is described in detail by the following preferred embodiments and the accompanying drawings.
Figure 1 is a schematic flowchart of one embodiment of method for monitoring software crash of the present invention.
Figure 2 is a schematic flowchart of another embodiment of the method for monitoring software crash of the present invention.
Figure 3 is a schematic circuit of the medical external equipment having a function of monitoring software crash of the present invention.
Figure 4 is an another schematic circuit of the medical external equipment having a function of monitoring software crash of the present invention.

### DESCRIPTION OF EMBODIMENTS

To make the objectives, technical solutions, and advantages of the present invention clearer, the following further describes the present invention in detail with reference to the accompanying drawings and embodiments.

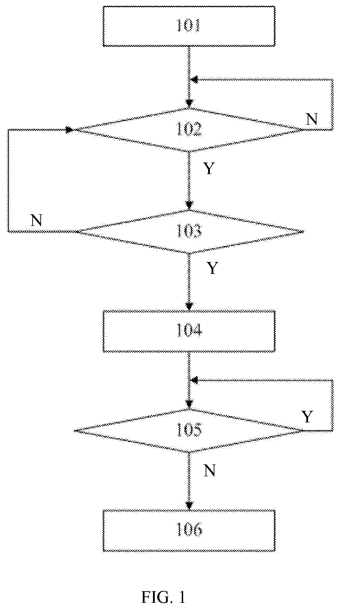

As shown in Figure 1, the method of assisting monitoring alarm of the present invention comprises the following steps:
step 101, selecting a medical host equipment for collecting and detecting patient's physiological parameters, and starting up a medical equipment host system;
wherein the medical equipment system may be a central monitoring system, a bedside monitoring equipment, and so on, the medical equipment system implemented by an software follows a communication protocol developed by an external equipment for monitoring software crash, and the external equipment for monitoring software crash may be used to makes crash monitoring,
step 102, judging whether there is an access from an external equipment by the medical equipment host system;
wherein, after started up, the medical equipment system detects whether there is an access from an external equipment for monitoring software crash at a specific external interface, the medical equipment system scans the external interface of the equipment at a certain interval to detect the access of the external equipment for monitoring software crash; if the access from an external equipment is detected, the detection is stopped. If there is no access from the equipment, the detection continues, that is, this step is repeated;
step 103, reading a preset identity information of the external equipment and judging whether the accessing external equipment is the external equipment for monitoring software crash by the medical equipment host system, if yes, going to step 4;
wherein when the medical equipment system scans and find the access from the external equipment, the medical equipment system judges whether the external equipment is matched; the detection method may comprise: reading some preset information of the equipment, such as protocol information, manufacturer information, serial number, equipment identification, etc.; if the equipment is matched, the detection is passed and the equipment is judged as the external equipment for monitoring software crash; if the equipment is not matched, the step 102 is repeated;
step 104, sending a predetermined protocol information to the external equipment for monitoring software crash in a predetermined time interval by the medical equipment host system;
wherein after having scanned and found the access from the external equipment, the medical equipment system sends the predetermined protocol information to the external equipment for monitoring software crash, the content of the protocol information as specific implementation are as follow:

**Table 1 the medical equipment system sending the predetermined protocol information**

| Byte | Content | Meaning | The number of byte occupied |
|---|---|---|---|
| byte 0 | 0xFF | Packet header | 1 |
| byte 1 | 4 (decimal) | Packet length | 1 |
| byte 2 | 0x01 | Module identification code | 1 |
| byte 3 | 0x06 | Frame type | 1 |
| byte 4 | *** | High byte of checksum | 1 |
| byte 5 | *** | Low byte of checksum | 1 |

### Note:

Checksum = sum of the values from byte (2) to byte (4+N);
High byte of checksum = (Checksum & 0xFF00) >>8;
Low byte of checksum = (Checksum & 0x00FF)
Packet length = the total number of bytes from module identification code (byte3) to low byte of checksum (byte (N+6))
step 105, determining whether the predetermined protocol information is received by the external equipment for monitoring software crash;
wherein the external equipment for monitoring software crash will receive the predetermined protocol information sent by the medical equipment host system, by determining whether the predetermined protocol information is received in the predetermined time interval, whether the medical equipment system crashes may be determined; if the external equipment for monitoring software crash receives the predetermined protocol information in the predetermined time interval, the method determines the medical equipment system operates normally, this determining step is repeated, if the external equipment for monitoring software crash can't receive the protocol information in time, the method determines the medical equipment system crashes and goes to step 106;
step 106, finding the medical equipment host system crashes and outputting a result by the external equipment for monitoring software crash;
wherein the external equipment for monitoring software crash finds the medical equipment host system crashes and outputs a result, preferably generates an alarm prompt, the crash alarm is preferably in the manner of alarm sound. The alarm reminds users to solve the problem of medical equipment system crash as soon as possible, to reduce the loss caused by the system crash, of course, the alarm prompt may be generated in the manner of the alarm sound and alarm light.

To make the objectives, technical solutions, and advantages of the present invention clearer, the following further describes the present invention in detail with reference to the accompanying drawings and embodiments. Another embodiment of the method for monitoring software crash of the present invention is provided.

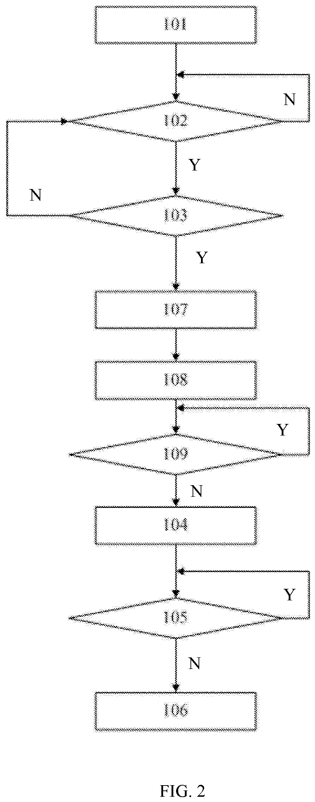

As shown in Figure 2, the specific steps of another embodiment of the method of assisting monitoring alarm of the present invention are described as follow:
After the step 103, the method further comprises:
step 107, performing self-check by the external equipment for monitoring software crash;
   when the external equipment for monitoring software crash accesses medical equipment system, the external equipment for monitoring software crash performs self-check, to detect whether the program and the hardware of the external equipment for monitoring software crash work normally,
step 108, simultaneously generating a prompt sound and/or prompt light indicating that the self-check is passed by the external equipment for monitoring software crash if the self-check is passed;
   It means that the external equipment for monitoring software crash works normally, the user can easily know the working status of the external equipment for monitoring software crash. After step 108, the method may go directly to step 104 (not shown) or to step 109.

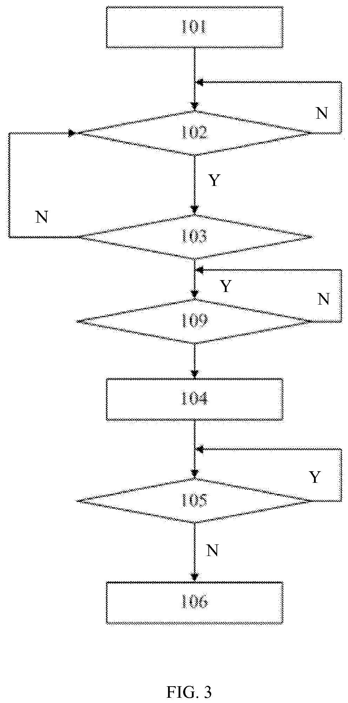

As shown in figures 2 and 3, after the step 103 or the step 108, the method further comprises: step 109, judging whether an information of activating a function of monitoring crash is received by the external equipment for monitoring software crash.

The medical equipment system may send an activating information to control whether the external equipment for monitoring software crash activates the function of monitoring crash. The external equipment for monitoring software crash activates the function of monitoring crash when receives the activating information; if no activating information is received, the step is repeated. Generally speaking, for integration of resources utilization, the external equipment for monitoring software crash has encryption dog, storage and other functions. By switching on a switch to control the function of monitoring crash, users can easily choose whether the function of monitoring crash is needed, thereby using the resources reasonably.

The content of the information of activating the function of monitoring crash as specific implementation are as follows:

**Table 2 medical equipment system sends the information of activating the function of monitoring crash**

| Byte | Content | (Meaning | The number of byte occupied |
|---|---|---|---|
| byte 0 | 0xFF | Packet header | 1 |
| byte 1 | 5 (decimal) | Packet length | 1 |
| byte 2 | 0x01 | Module identification code | 1 |
| byte 3 | 0x08 | Frame type | 1 |
| byte 4 | *** | 0x01 : switch on | 1 |
| | | 0x00 : switch off | |
| byte 5 | *** | High byte of checksum | 1 |
| byte 6 | *** | Low byte of checksum | 1 |

### Note:

Checksum = sum of the values from byte (2) to byte (4+N);
High byte of checksum = (Checksum & 0xFF00) >>8;
Low byte of checksum = (Checksum & 0x00FF)
Packet length = the total number of bytes from module identification code (byte3) to low byte of checksum (byte (N+6))

To make the objectives, technical solutions, and advantages of the present invention clearer, the following further describes the present invention in detail with reference to the accompanying drawings and embodiments.

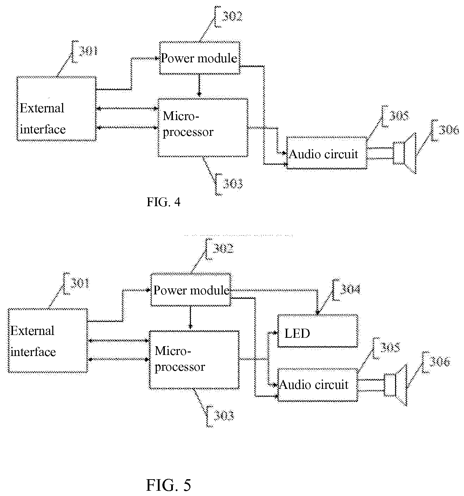

As shown in Figure 4, the schematic circuit of the medical external equipment having a function of monitoring crash of the present invention is described as follow, the medical external equipment comprises:
an external interface 301, configured to connect with an medical equipment host system for intercommunication;
wherein the general external interface of the external equipment for monitoring software crash (such as USB port, serial port, parallel port, PS2, Ethernet port and so on), is connected to the medical equipment system through the external interface 301, the external equipment for monitoring software crash may obtain power required by the external equipment for monitoring software crash through the external interface 301, and the external equipment for monitoring software crash may communicate with the medical equipment system through the external interface 301;
a power module 302, connected with the external interface 301 and configured to convert a voltage of the power supply from the external interface or provided by a DC power supply to the voltage suitable for the external equipment for monitoring software crash;
wherein the power module is connected to the external interface, a microprocessor and an audio circuit, the power module converts a voltage of the power supply from the external interface or provided by a DC power supply to the voltage suitable for different devices, and some interfaces that can't provide power may use the DC power supply such as a battery to provide power to the processor and the audio circuit module by converting the voltage to the required voltage by the power module;
a microprocessor 303, connected to the external interface 301 and the power module 302 and configured to receive and process a communication information from the external interface;
wherein the microprocessor is configured to receive the communication information, perform self-check to the external equipment for monitoring software crash and control the output of the audio and light signals;
an audio circuit 305, connected to the power module 302 and the microprocessor 303 and configured to receive a control information from the microprocessor and perform audio decoding control and output; and
a speaker 306, connected with the audio circuit 305 and configured to play an alarm prompt sound or play a self-check prompt sound.

As shown in Figure 5, another schematic circuit of the medical external equipment having a function of assisting monitoring alarm of the present invention is described as follow, the medical external equipment further comprises:
an LED module 304, connected to the power module 302 and the microprocessor 303 and configured to receive the control information from the microprocessor and display alarm light or prompt light.

In addition, in order to explain the invention better, the invention also provides a medical host equipment having a function of monitoring crash comprising:
an external interface connected with an external equipment for monitoring software crash for intercommunication;
an external equipment access judgment module, connected with the external interface and configured to judge whether there is an access from the external equipment;
an external equipment matching judgment module, connected with the external interface and configured to judge whether the accessing external equipment is the external equipment for monitoring software crash; and
a protocol information sending module, connected with the external interface and configured to send a predetermined protocol information to the external equipment for monitoring software crash in a predetermined time interval to the external equipment for monitoring software crash.

Further, the medical host equipment having the function of monitoring software crash also comprises:
crash monitoring activating module, connected with the external interface and configured to activate a communication information of crash monitoring.

The foregoing descriptions are merely exemplary embodiment of the present invention, but are not intended to limit the present invention to it. Any modification, equivalent replacement, or improvement made without departing from the scope and principle of the present invention shall fall within the protection scope of the present invention.

## Claims

1. A method for monitoring software crash, **characterized in that** the method comprises the following steps:
step 1, selecting a medical host equipment for collecting and detecting patient's physiological parameters, and starting up a medical equipment host system;
step 2, judging whether there is an access from a general external interface of a medical external equipment by the medical equipment host system, if yes, going to step 3;
step 3, communicating with the medical external equipment according to a protocol established by the medical host equipment to be monitored in the medical equipment system, reading a preset identity information of the external equipment and judging whether the medical external equipment requested for assessing the medical equipment host system is the medical external equipment for monitoring software crash by the medical equipment host system, if yes, going to step 4;
step 4, sending a predetermined protocol information to the medical external equipment for monitoring software crash in a predetermined time interval by the medical equipment host system;
step 5, determining whether the predetermined protocol information is received by the medical external equipment for monitoring software crash, if no, going to step 6;
step 6, finding the medical equipment host system crashes and outputting a result by the medical external equipment for monitoring software crash.

2. The method of claim 1, **characterized in that** the method further comprises a substep in step 6: generating an alarm sound and/or alarm light.

3. The method of claim 2, **characterized in that** the method further comprises the following steps after step 3:
step 31, performing a self-check by the medical external equipment for monitoring software crash;
step 32, simultaneously generating a prompt sound and/or prompt light indicating that the self-check is passed by the medical external equipment for monitoring software crash if the self-check is passed.

4. The method of claim 3, **characterized in that** the method further comprises the following step after step 32: judging whether an information of activating a function of monitoring crash is received by the medical external equipment for monitoring software crash.

5. The method of claim 1, **characterized in that** the method further comprises the following step after step 3: judging whether an information of activating a function of monitoring crash is received by the medical external equipment for monitoring software crash.

6. The method of claim 1, **characterized in that** the preset identity information in step 3 comprises a protocol information and/or a manufacturer information and/or a serial number and/or an equipment identification.

7. The method of claim 1, **characterized in that**,
step 2 further comprises repeating step 2 if there is no access from the medical external equipment;
step 3 further comprises returning to step 2 if the medical external equipment request for accessing the medical equipment host system is not the medical external equipment for monitoring software crash by the medical equipment host system; and
step 5 further comprises repeating step 5 if the predetermined protocol information is received by the external equipment for monitoring software crash.

8. A medical external equipment having a function of monitoring software crash mentioned in the method of claim 1, **characterized in that** the medical external equipment comprises:
a general external interface configured to connect with an medical equipment host system for intercommunication;
a power module, which is connected with the general external interface and configured to convert a voltage of the power supply from the external interface or provided by a DC power supply to the voltage suitable for the external equipment for monitoring software crash;
a microprocessor, which is connected to the external interface and the power module and configured to receive and process a communication information from the external interface;
an audio circuit, which is connected to the power module and the microprocessor and configured to receive a control information from the microprocessor and perform audio decoding control and output; and
a speaker, which is connected with the audio circuit and configured to play an alarm prompt sound or play a self-check prompt sound.

9. The medical external equipment having the function of monitoring software crash of claim 8, **characterized in that** the medical external equipment further comprises an LED module connected to the power module and the microprocessor and configured to receive the control information from the microprocessor and display alarm light or prompt light.

10. A medical host equipment having a function of monitoring software crash mentioned in the method of claim 1, **characterized in that** the medical host equipment comprises:
an external interface connected with a medical external equipment for monitoring software crash for intercommunication;
an external equipment access judgment module, which is connected with the external interface and configured to judge whether there is an access from the medical external equipment;
an external equipment matching judgment module, which is connected with the external interface and configured to judge whether the external equipment requested for accessing the medical equipment host system is the external equipment for monitoring software crash; and
a protocol information sending module, which is connected with the external interface and configured to send a predetermined protocol information to the medical external equipment for monitoring software crash in a predetermined time interval.

11. The medical host equipment of claim 10, **characterized in that** the medical host equipment further comprises a crash monitoring activating module, which is connected with the external interface and configured to activate communication information for crash monitoring.

## Patentansprüche

1. Verfahren zur Überwachung von Softwareabstürzen, **dadurch gekennzeichnet, dass** das Verfahren die folgenden Schritte umfasst:
Schritt 1 Auswählen einer medizinischen Hosteinrichtung zum Sammeln und Detektieren von physiologischen Parametern eines Patienten und Starten eines Medizinische-Einrichtung-Hostsystems;
Schritt 2 Beurteilen, ob es einen Zugriff von einer allgemeinen externen Schnittstelle einer externen medizinischen Einrichtung durch das Medizinische-Einrichtung-Hostsystem gibt, falls ja, Übergehen zu Schritt 3;
Schritt 3 Kommunizieren mit der externen medizinischen Einrichtung gemäß einem Protokoll, das von der zu überwachenden medizinischen Hosteinrichtung in dem medizinischen Einrichtungssystem erstellt wird, Lesen einer voreingestellten Identitätsinformation der externen Einrichtung und Beurteilen, ob die externe medizinische Einrichtung, die Zugriff auf das Medizinische-Einrichtung-Hostsystem fordert, die externe medizinische Einrichtung zum Überwachen von Softwareabstürzen durch das Medizinische-Einrichtung-Hostsystem ist, falls ja, Übergehen zu Schritt 4;
Schritt 4 Senden einer vorbestimmten Protokollinformation zu der externen medizinischen Einrichtung zum Überwachen von Softwareabstürzen in einem vorbestimmten Zeitintervall durch das Medizinische-Einrichtung-Hostsystem;
Schritt 5 Bestimmen, ob die vorbestimmte Protokollinformation von der externen medizinischen Einrichtung zum Überwachen von Softwareabstürzen empfangen wird, falls nein, Übergehen zu Schritt 6;
Schritt 6 Finden der Medizinische-Einrichtung-Hostsystem-Abstürze und Ausgeben eines Ergebnisses durch die externe medizinische Einrichtung zum Überwachen von Softwareabstürzen.

2. Verfahren nach Anspruch 1, **dadurch gekennzeichnet, dass** das Verfahren ferner einen Unterschritt in Schritt 6 umfasst: Erzeugen eines Alarmtons und/oder eines Alarmlichts.

3. Verfahren nach Anspruch 2, **dadurch gekennzeichnet, dass** das Verfahren ferner den folgenden Schritt nach Schritt 3 umfasst:
Schritt 31 Durchführen einer Selbstprüfung durch die externe medizinische Einrichtung zum Überwachen von Softwareabstürzen;
Schritt 32 gleichzeitiges Erzeugen eines Auslösetons und/oder eines Auslöselichts, der/das anzeigt, dass die Selbstprüfung von der externen medizinischen Einrichtung zum Überwachen von Softwareabstürzen durchgeführt worden ist, falls die Selbstprüfung durchgeführt worden ist.

4. Verfahren nach Anspruch 3, **dadurch gekennzeichnet, dass** das Verfahren ferner den folgenden Schritt nach Schritt 32 umfasst: Beurteilen, ob eine Information einer Aktivierung einer Funktion des Überwachens von Abstürzen von der externen medizinischen Einrichtung zum Überwachen von Softwareabstürzen empfangen wird.

5. Verfahren nach Anspruch 1, **dadurch gekennzeichnet, dass** das Verfahren ferner den folgenden Schritt nach Schritt 3 umfasst: Beurteilen, ob eine Information einer Aktivierung einer Funktion des Überwachens von Abstürzen von der externen medizinischen Einrichtung zum Überwachen von Softwareabstürzen empfangen wird.

6. Verfahren nach Anspruch 1, **dadurch gekennzeichnet, dass** die voreingestellte Identitätsinformation in Schritt 3 eine Protokollinformation und/oder eine Herstellerinformation und/oder eine Seriennummer und/oder eine Einrichtungsidentifikation umfasst.

7. Verfahren nach Anspruch 1, **dadurch gekennzeichnet, dass**
Schritt 2 ferner das Wiederholen von Schritt 2 umfasst, falls es keinen Zugriff von der externen medizinischen Einrichtung gibt;
Schritt 3 ferner das Zurückkehren zu Schritt 2 umfasst, falls die externe medizinische Einrichtung, die das Zugreifen auf das Medizinische-Einrichtung-Hostsystem anfordert, nicht die externe medizinische Einrichtung zum Überwachen von Softwareabstürzen durch das Medizinische-Einrichtung-Hostsystem ist; und
Schritt 5 ferner das Wiederholen von Schritt 5 umfasst, falls die vorbestimmte Protokollinformation von der externen Einrichtung zum Überwachen von Softwareabstürzen empfangen wird.

8. Externe medizinische Einrichtung mit einer Funktion zum Überwachen von Softwareabstürzen gemäß dem Verfahren nach Anspruch 1, **dadurch gekennzeichnet, dass** die externe medizinische Einrichtung umfasst:
eine allgemeine externe Schnittstelle zum Verbinden mit einem Medizinische-Einrichtung-Hostsystem für eine Interkommunikation;
ein Leistungsmodul, das mit der allgemeinen externen Schnittstelle verbunden ist und derart ausgeführt ist, dass es eine Spannung der Leistungsversorgung von der externen Schnittstelle, oder die von einer DC-Leistungsversorgung bereitgestellt wird, in die Spannung, die für die externe Einrichtung zum Überwachen von Softwareabstürzen geeignet ist, konvertiert;
einen Mikroprozessor, der mit der externen Schnittstelle und dem Leistungsmodul verbunden ist und derart ausgeführt ist, dass er eine Kommunikationsinformation von der externen Schnittstelle empfängt und verarbeitet;
eine Audioschaltung, die mit dem Leistungsmodul und dem Mikroprozessor verbunden ist und derart ausgeführt ist, dass sie eine Steuerinformation von dem Mikroprozessor empfängt und eine Audiodekodierungssteuerung und - ausgabe durchführt; und
einen Lautsprecher, der mit der Audioschaltung verbunden ist und derart ausgeführt ist, dass er einen Alarmauslöseton abspielt oder einen Selbstprüfungsauslöseton abspielt.

9. Externe medizinische Einrichtung mit der Funktion des Überwachens von Softwareabstürzen nach Anspruch 8, **dadurch gekennzeichnet, dass** die externe medizinische Einrichtung ferner ein LED-Modul aufweist, das mit dem Leistungsmodul und dem Mikroprozessor verbunden ist und derart ausgeführt ist, dass es die Steuerinformation aus dem Mikroprozessor empfängt und ein Alarmlicht oder Auslöselicht anzeigt.

10. Medizinische Hosteinrichtung mit der Funktion des Überwachens von Softwareabstürzen nach Anspruch 1, **dadurch gekennzeichnet, dass** die medizinische Hosteinrichtung umfasst:
eine externe Schnittstelle, die zur Interkommunikation mit einer externen medizinischen Einrichtung zum Überwachen von Softwareabstürzen verbunden ist;
ein Externe-Einrichtung-Zugriffsbeurteilungsmodul, das mit der externen Schnittstelle verbunden ist und derart ausgeführt ist, dass es beurteilt, ob es einen Zugriff von der externen medizinischen Einrichtung gibt;
ein Externe-Einrichtung-Übereinstimmungsbeurteilungsmodul, das mit der externen Schnittstelle verbunden ist und derart ausgeführt ist, dass es beurteilt, ob die externe Einrichtung, die einen Zugriff auf das Medizinische-Einrichtung-Hostsystem anfordert, die externe Einrichtung zum Überwachen von Softwareabstürzen ist; und
ein Protokollinformations-Sendemodul, das mit der externen Schnittstelle verbunden ist und derart ausgeführt ist, dass es in einem vorbestimmten Zeitintervall eine vorbestimmte Protokollinformation an die externe medizinische Einrichtung zum Überwachen von Softwareabstürzen sendet.

11. Medizinische Hosteinrichtung nach Anspruch 10, **dadurch gekennzeichnet, dass** die medizinische Hosteinrichtung ferner ein Abstürzüberwachungs-Aktivierungsmodul umfasst, das mit der externen Schnittstelle verbunden ist und derart ausgeführt ist, dass es eine Kommunikationsinformation zur Abstürzüberwachung aktiviert.

## Revendications

1. Procédé de surveillance de plantage de logiciel, **caractérisé en ce que** le procédé comprend les étapes suivantes :
étape 1, la sélection d'un équipement hôte médical pour recueillir et détecter des paramètres physiologiques d'un patient, et la mise en route d'un système hôte d'équipement médical ;
étape 2, le fait d'estimer s'il y a un accès depuis une interface externe générale d'un équipement externe médical par le système hôte d'équipement médical, si oui, aller à l'étape 3 ;
étape 3, la communication avec l'équipement externe médical selon un protocole établi par l'équipement hôte médical à surveiller dans le système d'équipement médical, la lecture d'une information d'identité préétablie de l'équipement externe et le fait d'estimer si l'équipement externe médical demandé pour accéder au système hôte d'équipement médical est l'équipement externe médical pour surveiller un plantage de logiciel par le système hôte d'équipement médical, si oui, aller à l'étape 4 ;
étape 4, l'envoi d'une information de protocole prédéterminée à l'équipement externe médical pour surveiller un plantage de logiciel dans un intervalle de temps prédéterminé par le système hôte d'équipement médical ;
étape 5, le fait de déterminer si l'information de protocole prédéterminée est reçue par l'équipement externe médical pour surveiller un plantage de logiciel, si non, aller à l'étape 6 ;
étape 6, la découverte que le système hôte d'équipement médical plante et la fourniture en sortie d'un résultat par l'équipement externe médical pour surveiller un plantage de logiciel.

2. Procédé selon la revendication 1, **caractérisé en ce que** le procédé comprend en outre une sous-étape à l'étape 6 : la génération d'un son d'alarme et/ou d'une lumière d'alarme.

3. Procédé selon la revendication 2, **caractérisé en ce que** le procédé comprend en outre les étapes suivantes après l'étape 3 :
étape 31, la réalisation d'une auto-vérification par l'équipement externe médical pour surveiller un plantage de logiciel ;
étape 32, la génération simultanée d'un son d'invite et/ou d'une lumière d'invite indiquant que l'auto-vérification est réussie par l'équipement externe médical pour surveiller un plantage de logiciel si l'auto-vérification est réussie.

4. Procédé selon la revendication 3, **caractérisé en ce que** le procédé comprend en outre l'étape suivante après l'étape 32 : le fait d'estimer si une information d'activation d'une fonction de surveillance de plantage est reçue par l'équipement externe médical pour surveiller un plantage de logiciel.

5. Procédé selon la revendication 1, **caractérisé en ce que** le procédé comprend en outre l'étape suivante après l'étape 3 : le fait d'estimer si une information d'activation d'une fonction de surveillance de plantage est reçue par l'équipement externe médical pour surveiller un plantage de logiciel.

6. Procédé selon la revendication 1, **caractérisé en ce que** l'information d'identité préétablie à l'étape 3 comprend une information de protocole et/ou une information de fabricant et/ou un numéro de série et/ou une identification d'équipement.

7. Procédé selon la revendication 1, **caractérisé en ce que**,
l'étape 2 comprend en outre la répétition de l'étape 2 s'il n'y a pas d'accès depuis l'équipement externe médical ;
l'étape 3 comprend en outre le retour à l'étape 2 si la demande de l'équipement externe médical d'accéder au système hôte d'équipement médical n'est pas l'équipement externe médical pour surveiller un plantage de logiciel par le système hôte d'équipement médical ; et
l'étape 5 comprend en outre la répétition de l'étape 5 si l'information de protocole prédéterminée est reçue par l'équipement externe pour surveiller un plantage de logiciel.

8. Equipement externe médical ayant une fonction de surveillance de plantage de logiciel mentionné dans le procédé de la revendication 1, **caractérisé en ce que** l'équipement externe médical comprend :
une interface externe générale configurée pour se connecter à un système hôte d'équipement médical pour une intercommunication ;
un module d'alimentation, qui est connecté à l'interface externe générale et configuré pour convertir une tension de l'alimentation électrique en provenance de l'interface externe ou fourni par une alimentation électrique continue en la tension adaptée pour l'équipement externe pour surveiller un plantage de logiciel ;
un microprocesseur, qui est connecté à l'interface externe et au module d'alimentation et configuré pour recevoir et traiter une information de communication provenant de l'interface externe ;
un circuit audio, qui est connecté au module d'alimentation et au microprocesseur et configuré pour recevoir une information de commande en provenance du microprocesseur et réaliser une commande et une sortie de décodage audio ; et
un haut-parleur, qui est connecté au circuit audio et configuré pour lire un son d'invite d'alarme ou lire un son d'invite d'auto-vérification.

9. Equipement externe médical doté de la fonction de surveillance de plantage de logiciel de la revendication 8, **caractérisé en ce que** l'équipement externe médical comprend en outre un module de DEL connecté au module d'alimentation et au microprocesseur et configuré pour recevoir l'information de commande en provenance du microprocesseur et afficher une lumière d'alarme ou une lumière d'invite.

10. Equipement hôte médical doté d'une fonction de surveillance de plantage de logiciel mentionné dans le procédé de la revendication 1, **caractérisé en ce que** l'équipement hôte médical comprend :
une interface externe connectée à un équipement externe médical pour surveiller un plantage de logiciel pour une intercommunication ;
un module d'estimation d'accès d'équipement externe, qui est connecté à l'interface externe et configuré pour estimer s'il y a un accès depuis l'équipement externe médical ;
un module d'estimation de concordance d'équipement externe, qui est connecté à l'interface externe et configuré pour estimer si l'équipement externe demandé pour accéder au système hôte d'équipement médical est l'équipement externe pour surveiller un plantage de logiciel ; et
un module d'envoi d'information de protocole, qui est connecté à l'interface externe et configuré pour envoyer une information de protocole prédéterminée à l'équipement externe médical pour surveiller un plantage de logiciel dans un intervalle de temps prédéterminé.

11. Equipement hôte médical selon la revendication 10, **caractérisé en ce que** l'équipement hôte médical comprend en outre un module d'activation de surveillance de plantage, qui est connecté à l'interface externe et configuré pour activer une information de communication pour la surveillance de plantage.
